# EUROPÄISCHE PATENTSCHRIFT

(11) **EP 2 825 264 B1**
(45) Veröffentlichungstag und Bekanntmachung des Hinweises auf die Patenterteilung: **03.05.2017**
(21) Anmeldenummer: 13701280.3
(22) Anmeldetag: 28.01.2013
(51) Int. Cl.: A61Q 5/10, A61K 8/33, A61K 8/34, A61K 8/37, A61Q 5/08, A61K 8/891, A61K 8/73, A61K 8/92, A61K 8/31

(54) **OXIDATIONSMITTELZUBEREITUNG MIT OPTIMIERTER VISKOSITÄT ZUR BEHANDLUNG VON KERATINISCHEN FASERN**
OXIDANT PREPARATION HAVING OPTIMIZED VISCOSITY FOR TREATING KERATIN FIBERS
PRÉPARATION D'OXYDANT À VISCOSITÉ OPTIMISÉE POUR LE TRAITEMENT DE FIBRES KÉRATINIQUES

(30) Priorität: 31.01.2012 DE 102012201338
(43) Veröffentlichungstag der Anmeldung: 21.01.2015
(73) Patentinhaber: Henkel AG & Co. KGaA, 40589 Düsseldorf (DE)
(72) Erfinder: GOUTSIS, Konstantin, 41363 Jüchen (DE); SCHÖPGENS, Jürgen, 41366 Schwalmtal (DE); KRIPPAHL,Marc, 41239 Mönchengladbach (DE)
(86) Internationale Anmeldenummer: PCT/EP2013/051541
(87) Internationale Veröffentlichungsnummer: WO 2013/113645

(56) Entgegenhaltungen:
- WO-A2-2010/062138
- DE-A1-102010 003 264
- US-A- 4 904 275
- US-A1- 2002 139 957
- US-A1- 2010 307 527
- US-A1- 2011 067 722
- DATABASE GNPD [Online] MINTEL; 1. Januar 2008 (2008-01-01), "Natural Bleach & Colour", XP002731269, Database accession no. 849228
- DATABASE GNPD [Online] MINTEL; 1. November 2011 (2011-11-01), "Highlights Hair Colour", XP002731270, Database accession no. 1666753
- DATABASE GNPD [Online] MINTEL; 1. Juli 2005 (2005-07-01), "Hydrating Gentle Facial Cleanser", XP002731271, Database accession no. 10224698
- DATABASE GNPD [Online] MINTEL; 1. September 2009 (2009-09-01), "Natural Bleach Colour", XP002731272, Database accession no. 1203289
- DATABASE GNPD [Online] MINTEL; 1. November 2007 (2007-11-01), "Medium Brown Hair Colorant", XP002731273, Database accession no. 811029

## Beschreibung

Gegenstand der vorliegenden Anmeldung sind Oxidationsmittelzubereitungen zum Behandeln von keratinischen Fasern, insbesondere menschlichen Haaren, welche jeweils in speziellen Mengenbereichen Wasserstoffperoxid, mindestens eine lipophile Verbindung und als Verdicker mindestens ein Polysaccharid enthalten. Ein Verfahren zum Anmischen und Auftragen anwendungsbereiter Formulierungen zur Farbveränderung von keratinischen Fasern unter Einsatz der Oxidationsmittelzubereitungen ist ebenfalls Gegenstand der vorliegenden Anmeldung.

Zur Bereitstellung farbverändernder kosmetischer Mittel, insbesondere für keratinische Fasern, wie beispielsweise Haare, kennt der Fachmann je nach Anforderung an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Die Oxidationsfärbemittel zeichnen sich durch hervorragende, lang anhaltende Färbeergebnisse aus. Neben der Färbung ist auch das Aufhellen bzw. Blondieren der eigenen Haarfarbe der ganz spezielle Wunsch vieler Verbraucher. Dazu werden die die Faser färbenden natürlichen oder künstlichen Farbstoffe meist oxidativ unter Einsatz von entsprechenden Oxidationsmitteln, wie beispielsweise Wasserstoffperoxid, entfärbt.

Um eine optimale Färbe- und Aufhellleistung zu entfalten, benötigen oxidative Färbe- bzw. Aufhellmittel bei der Anwendung in der Regel einen alkalischen pH-Wert, insbesondere bei pH-Werten zwischen 8,5 und 10,5 werden optimale Ergebnisse erzielt.

Sowohl bei der oxidativen Färbung als auch bei der Aufhellung bzw. Blondierung von keratinischen Fasern wird standardmäßig Wasserstoffperoxid als Oxidationsmittel eingesetzt. Wässrige Wasserstoffperoxidlösungen sind bei den für die Anwendung benötigten alkalischen pH-Werten jedoch instabil, so dass handelsübliche oxidative Färbe- und Biondierprodukte in der Regel aus mindestens zwei Komponenten bestehen. Bei der ersten Komponente handelt es sich um eine sauer eingestellte Oxidationsmittelzubereitung mil Wasserstoffperoxid, welche kurz vor der Anwendung mit einer alkalisch eingestellten zweiten Komponente, die beispielsweise in Form einer Creme vorliegt, vermischt wird. Im Fall eines oxidativen Färbeproduktes enthält diese zweite Komponente zusätzlich Oxidationsfarbstoffvorprodukte vom Entwickler und/oder Kupplertyp.

Die Vermischung beider Komponenten kann unter Zuhilfenahme verschiedener Mischvorrichtungen vorgenommen werden. Eine vornehmlich im Heimanwenderbereich etablierte Methode des Vermischens besteht darin, beide Komponenten in eine Applikatorflasche zu überführen, diese durch Schütteln der Flasche zu einer homogenen Anwendungsformulierung zu vermischen und anschließend über eine Öffnung der Applikatorflasche auf die Keratinfasern aufzutragen. Bei einer weiteren, oft vom Friseur vorgenommenen Methode des Vermischens werden beide Komponenten in eine Anmischschale gegeben, wo sie nach dem sorgfältigen Durchrühren bzw. Vermengen unter Zuhilfenahme eines Pinsel oder einer Applicette auf das Haar aufgetragen werden.

Beide Methoden stellen spezielle Anforderungen an die Konfektionierung und die Viskosität der jeweils eingesetzten Formulierungen, wobei sowohl die Viskositäten der Einzelkomponenten (Oxidationsmittelzubereitung und alkalische (Creme)Komponente) als auch die Viskosität der finalen Anwendungsmischung für die Erzielung eines gleichmäßigen und intensiven Färbe- bzw. Aufhellergebnisses maßgeblich sind.

Werden bei dem Mischvorgang in einer Anmischschale Einzelformulierungen miteinander vermischt, deren Viskositäten sich zu stark unterscheiden, ist die Vermengung zu einer homogenen Anwendungsformulierung nur schwer bzw. erst nach langem Rühren möglich. Die Viskositäten von Oxidationsmittelzubereitung und alkalischer (Creme)Komponente müssen daher genau aufeinander abgestimmt sein. Insbesondere wenn eine oder beide der eingesetzten Formulierungen eine zu hohe Viskosität aufweisen und damit viel zu dickflüssig sind, kann die vollständige Durchmischung durch Rühren mit einem Pinsel nur schwer erzielt werden.

Weiterhin muss die optimale Entnehmbarkeit der Oxidationsmitteizubereitung aus dem Behältnis, in dem sie für die Verwendung bereitgestellt wird, gewährleistet sein. Bei diesem Behältnis handelt es sich meist um eine flexible Flasche mit einer kleinen Öffnung, aus der die Oxidationsmittelzubereitung durch Drücken der Flasche entnommen werden kann. Oxidationsmittelzubereitungen mit zu hoher Viskosität lassen sich nur langsam, unvollständig und nur durch hohen Druck auf die Flasche entnehmen.

Dünnflüssige Formulierungen lassen sich besser aus der Flasche in die Anmischschale überführen, weisen jedoch bei der anschließenden Vermischung mit mit der meist cremeförmigen, höherviskosen zweiten Komponente Nachteile auf.

Die Viskositäten von Formulierungen, die allen vorgenannten Anforderungen genügen sollen, müssen daher zuverlässig in einem definierten Bereich liegen, welcher nur wenig Variationsbreite in Richtung zu hoher oder zu niedriger Viskositäten zulässt.

Die Viskosität von Formulierungen kann durch Einsatz von polymeren Verdickern eingestellt werden. Soll die Viskosität von Oxidationsmittel beinhaltenden Formulierungen reguliert werden, so müssen die eingesetzten Verdicker zusätzlich gegenüber den Oxidationsmitteln stabil sein.

Für Oxidationsmittelformulierungen mit Wasserstoffperoxid haben sich bislang Homo- und Copolymere der Acrylsäure oder der Methacrylsäure als das Verdickersystem der Wahl erwiesen. Acrylsäuerpolymere und entsprechende Derivate besitzen eine hohe Stabilität gegenüber Wasserstoffperoxid, verdicken zuverlässig gerade im alkalischen Milieu und sind kompatibel mit Elektrolyten, Salzen und polaren Lösungsmitteln.

Unvernetzte oder vernetzte Polyacrylsäure-Polymere werden beispielsweise in der DE 10 2010 003 263 A1 und in der DE 37 32 147 A1 zur Verdickung von Oxidationsmittelzubereitungen eingesetzt. Bei den in der DE 10 2010 003 264 A1 beschriebenen Oxidationsmittelzubereitungen handelt es sich um zweiphasige Systeme, die zur Einstellung der Viskosität ebenfalls Polyacrylsäure-Polymere enthalten. Infolge ihrer schlechten biologischen Abbaubarkeit gibt es nun aber zunehmend Bestrebungen,
die Einsatzmenge von Acrylsäurepolymeren in Kosmetikprodukten zu reduzieren oder sie durch natürliche Verdicker oder Quellmittel mit verbessertem ökologischem Profil komplett zu ersetzen. Natürliche Quellmittel besitzen jedoch den Nachteil, dass sie, wenn sie zu hoch dosiert werden, auf der Haut leicht einen Film bilden können. Derartige Rückstände sind vom Konsumenten nicht erwünscht.

Wird die Einsatzkonzentration der biologischen Quellmittel dagegen so stark verringert, dass nach der Anwendung keine Rückstände auf der Haut zurückbleiben, besteht die Gefahr, dass sich die Viskosität nicht zuverlässig über längere Lagerzeit auf den gewünschten Wert einstellen lässt bzw. die Formulierungen zu dünn bleiben.

US 2011/0067722 A1 offenbart mit den Beispielen 1 bis 10 Haarbleichmittel, enthaltend 1,65 Gew.-% Cetearylalkohol (lipophile Verbindung), Xanthan Gum (Polysaccharid) in variierenden Gewichtsanteilen und Wasserstoffperoxid.

EP 1803436 A1 offenbart mit dem Beispiel 1 eine anwendungsbereite Bleichmittelformulierung, enthaltend 6,0 Gew.-% Wasserstoffperoxid, 1,13 Gew.-% Cetearylalkohol (lipophile Verbindung), 0,1 Gew.-% Xanthan, 1,06 Gew.-% Cellulose, 0,46 Gew.-% Hydroxyethylcellulose und 9,67 Gew.-% Di(ethylhexyl)carbonat).

DE 19962877 A1 adressiert die Konditionierwirkungen vom Mitteln auf Haut und Haaren. Unter die Konditionierwirkung fallen beispielsweise die Glätte, der Glanz und die Kämmbarkeit von Haaren. Erzielt werden sollen diese Effekte durch den Einsatz einer Kombiantion aus Dialkylcarbonaten und Aminoalkylamiden.

Es war daher die Aufgabe der vorliegenden Erfindung, Oxidationsmittelzubereitungen zur Verfügung zu stellen, welche den Verzicht auf synthetische Polymere - insbesondere vom Polyacrylsäuretyp - als Formulierungsbestandteil ermöglichen. Die Viskosität dieser Formulierungen soll sich hierbei zuverlässig so einstellen lassen, dass die optimale Durchmischbarkeit mit einer alkalischen (Creme)Komponente gewährleistet ist. Weiterhin soll sich die Oxidationsmittelzubereitung komplett aus dem Behältnis, in dem sie zur Verfügung gestellt wird, entnehmen lassen.

Nach dem Durchmischen soll die Anwendungsformulierung eine Viskosität besitzen, die die komfortable Applikation der Formulierung auf die Keratinfasern ermöglicht, wobei sich die Anwendungsformulierung gut verteilen lassen, zu einer gleichmäßigen Farbänderung führen, dabei aber gleichzeitig nicht vom Kopf der Konsumenten heruntertropfen soll. Darüber hinaus sollen alle zur Einstellung der Viskosität eingesetzten Verdicker biologisch abbaubar sein und nach der Anwendung nicht in Form von Rückständen auf der Haut des Konsumenten zurückbleiben.

Überraschenderweise hat sich im Zuge der zu dieser Erfindung führenden Arbeiten herausgestellt, dass es möglich ist, die Viskosität von Oxidationsmittelzubereitungen mit Wasserstoffperoxid ohne die oben beschriebenen Nachteile zuverlässig auf den gewünschten Spezifikationsbereich einzustellen, wenn hierzu eine spezielle Fettbasis (organisch lipophile Verbindung) und geringe Mengen eines biologisch abbaubaren Polymers eingesetzt werden.

Ein erster Gegenstand der vorliegenden Erfindung ist daher eine Oxidationsmittelzubereitung zur Behandlung von keratinischen Fasern, insbesondere von menschlichen Haaren, enthaltend in einem kosmetischen Träger
(a) 0,01 bis 25,0 Gew.-% Wasserstoffperoxid,
(b) 0,01 bis 15,0 Gew.-% einer oder mehrerer organisch lipophiler Verbindung(en) aus der Gruppe der pflanzlichen und tierischen Fette, Öle und Wachse, der Paraffinkohlenwasserstoffe, der C₁₀-C₂₄-Fettalkohole, der Silikonöle und der Dialkylether der Formel (I),

   R1-O-R2 (I)

   worin
   R1 und R2 unabhängig voneinander für eine unverzweigte oder verzweigte C₈-C₂₄-Alkylgruppe stehen, wobei die Oxidationsmittelzubereitung 5,0 bis 14,0 Gew.-% Paraffinkohlenwasserstoffe enthält, und
(c) 0,01 bis 2,0 Gew.-% eines oder mehrerer organischer Verdicker aus der Gruppe der Polysaccharide, wobei die Oxidationsmittelzubereitung 0,1 bis 1,0 Gew.-% Xanthan Gum enthält, und
(d) 0,1 bis 10,0 Gew.-% mindestens eines organischen Carbonats ausgewählt aus Glycerincarbonat, Propylencarbonat, Ethylencarbonat, Dimethylcarbonat, Diethylcarbonat, Di-n-hexylcarbonat, Di-n-octyl-carbonat, Di-n-decylcarbonat und Di-n-dodecylcarbonat, mit der Maßgabe, dass die Oxidationsmlttelzubereitung
   - kein Polymer enthält, das durch Polymerisation oder Copolymerisation von Acrylsäure erhalten wird,
   - kein Polymer enthält, das durch Polymerisation oder Copolymerisation von Methacrylsäure erhalten wird,
   - kein Polymer enthält, das durch Polymerisation oder Copolymerisation von Acrylsäuerestern erhalten wird,
   - kein Polymer enthält, das durch Polymerisation oder Copolymerisation von Methacrylsäureestern erhalten wird.

Unter keratinhaltigen Fasern werden prinzipiell alle tierischen Haare, z. B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare. Unter dem erfindungsgemäß verwendeten Begriff "Behandeln von Keratinfasern" wird jede kosmetische Behandlung von keratinischen Fasern verstanden Insbesondere umfasst dieser Begriff die Farbveränderung der Fasern. Die Farbveränderung von Keratinfasern umfasst jedwede Form der Farbveränderung der Fasern. Umfasst sind insbesondere die unter den Begriffen Tönung, Aufhellung, Blondierung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Die erfindungsgemäßen Oxidationsmittelzubereitungen enthalten die erfindungswesentlichen Bestandteile in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Als ersten wesentlichen Formulierungsbestandteil (a) enthalten die erfindungsgemäßen Oxidationsmittelzubereitungen 0,01 bis 25,0 Gew.-% Wasserstoffperoxid.

Hierbei kann das Wasserstoffperoxid selbst als wässrige Lösung und/oder in Form eines seiner festen Anlagerungsprodukte an organische oder anorganische Verbindungen eingesetzt werden. Berechnungsgrundlage für den Mengenbereich von 0,01 bis 25,0 Gew.-% ist hierbei 100 %-iges Wasserstoffperoxid.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; erfindungsgemäß bevorzugte Oxidationsmittelzubereitungen sind dadurch gekennzeichnet, dass sie 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges H₂O₂) enthalten.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden.

Erfingdungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw deren Natriumsalze.

Als zweiten wesentlichen Formulierungsbestandteil (b) enthalten die erfindungsgemäßen Oxidationsmittelzubereitungen 0,01 bis 15,0 Gew.-% einer oder mehrerer organisch lipophiler Verbindung(en) aus der Gruppe der pflanzlichen und tierischen Fette, Öle und Wachse, der Paraffinkohlenwasserstoffe, der C₁₀-C₂₄-Fettalkohole, der Silikonöle und der Dialkylether der Formel (I),

R1-O-R2 (I)

worin
R1 und R2 unabhängig voneinander für eine unverzeigle oder verzweigte C₈-C₂₄-Alkylgruppe stehen, wobei die Oxidationsmittelzubereitung 5,0 bis 14,0 Gew.-% Paraffinkohlenwasserstoffe enthält,
Unter der Definition "organisch lipophile Verbindungen" wurden im Sinne der vorliegenden Erfindung alle geeigneten organischen, fettliebenden Verbindungen zusammengefasst, die unter Normalbedingungen (22 °C, Normaldruck) nicht oder nur wenig in Wasser löslich sind und daher in emulgierter bzw. dispergierter Form in der Oxidationsmittelzubereitung vorliegen.

Als pflanzliche und tierische Fette und Öle werden feste, halbfeste oder flüssige Produkte des Pflanzen- oder Tierkörpers verstanden, die im wesentlichen aus Triacylglyerolen (Fettsäuretriglyceriden) höherer Fettsäuren bestehen.

Unter einem Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₈-C₂₄-Carbonsäuren zu verstehen. Ungesättigte Fettsäuren können einfach oder mehrfach ungesättigt sein. Bei einer ungesättigten Fettsaure kann bzw. können deren C-C-Doppelbindung(en) die Cis- oder Trans-Konfiguration aufweisen.

Als pflanzliche oder tierische Wachse im Sinne der vorliegenden Erfindung werden Produkte des Pflanze- oder Tierkörpers verstanden, die im wesentlichen aus den Estern langkettiger Fettsäuren (C₂₄-C₃₆-Fottsäuren, auch als Wachsäuren bezeichnet) mit langkettigen Alkoholen (C₁₅-C₃₈-Fettalkoholen) bestehen,

Innerhalb der Gruppe der organisch lipophilen Verbindungen (b) sind die pflanzlichen Öle und Fette bevorzugt. Die Viskosität von Oxidationsmittelzubereitungen, die auf Basis von emulgierten bzw. dispergierten pflanzlichen Ölen und Fetten formuliert werden, lässt sich bereits durch Einsatz besonders kleiner Mengen an biologisch abbaubarem Polysaccharid reproduzierbar auf den gewünschten Wertebereich einstellen.

In einer bevorzugten Ausführungsform der vorliegenden Erfindung enthält die Oxidationsmittelzubereitung als organisch lipophile Verbindung (b) daher ein planzliches Fett und/oder ein pflanzliches Öl.

Die in Sojaöl, Erdnußöl, Olivenöl, Sonnenblumenöl, Macadamianussöl, Moringaöl, Aprikosenkernöl, Marulaöl, Kokosfett und/oder gegebenenfalls gehärtetem Rizinusöl vorkommenden Fettsäuretriglyceride bzw. Triglyceridgemische haben sich als besonders geeignet zum Einsatz als Fettkörper (organisch lipophile Verbindung) in den erfindungsgemäßen Oxidationsmittelzubereitungen erwiesen.

Sojaöl enthält ein Gemisch aus Triglyceriden, welches sich aus den Bestandteilen Palmitinsäure Stearinsäure, Ölsäure, Linolsäure und Linolensäure zusammensetzt. Erdnußöl setzt sich hauptsächlich aus den Bestandteilen Palmitinsäure, Stearinsäure, Behensäure, Lignocerinsäure, Ölsäure und Linolsäure zusammen, welche mit Glycerin verestert vorliegen. Olivenöl enthält hauptsächlich aus den Bestandteilen Myristinsäure, Palmitinsäure, Ölsäure und Linolsäure aufgebaute Fettsäuretriglyceride. Sonnenblumenöl beinhaltet hauptsächlich Trifettsäureglyceride auf Basis von Palmitinsäure, Stearinsäure, Ölsäure und Linolsäure. Bei den Trifettsäureglyceriden des Macadamianussöls bilden hauptsächlich die Fettsäuren Linolsäure, Palmitinsäure, Arachinsäure, Stearinsäure die Esterbindungen mit Glycerin aus. In Moringaöl ist ein Gemisch aus Fettsäuretriglyceriden enthalten,
welches durch Veresterung von Glycerin mit den Fettsäuren Myristinsäure, Palmitinsäure, Stearinsäure, Behensäure, Ölsäure und Linolsäure erhalten wird. Aprikosenkernöl beinhaltet als Hauptbestandteile Triglyceride mit den Fettsäuren Ölsäure und Linolsäure. Die Triglyceride des Kokosfetts setzen sich hauptsächlich aus den Fettsäuren Dodecansäure, Tetradocansäure, Ölsäure, Palmitinsäure, Decansäure und Octansäure und Stearinsäure zusammen. Marulaöl beinhaltet im wesentlichen Fettsäuretriglyceride aus Glycerin und Palmitinsäure, Stearinsäure, Ölsäure und Linolsäure,

Aus der vorgenannten Gruppe hat sich insbesondere der Einsatz eines Gemisches aus Fettsäuretriglyceriden, wie es in Kokosfett vorkommt, als besonders vorteilhaft bei der Einstellung der gewünschten Viskosität der erfindungsgemäßen Oxidationsmittelzubereitungen herausgestellt.

Als organisch lipophile Verbindung(en) (b) werden Paraffinkohlenwasserstoffe eingesetzt. Paraffinkohlenwasserstoffe sind ein festes oder flüssiges Gemisch gesättigter, aliphatischer Kohlenwasserstoffe. Unter diese Definition fällt das dickflüssige Paraffinum liquidum, das dünnflüssigere Paraffinum perliquidum und das Hartparaffin (Paraffinum solidum). Paraffinkohlenwasserstoffe werden auch als Paraffine, Paraffinöle oder Paraffinwachse bezeichnet.

Oxidationsmittelzubereitungen, die Paraffinkohlenwasserstoffe enthalten, lassen sich besonders gut bereits mit kleinen Mengen an Polysacchariden verdicken, ohne dass hierbei unerwünschte oder unvorhersehbare Effekte auftreten oder die Viskosität der Formulierung sich im Laufe der Zeit verändert. Der Einsatz von Paraffinkohlenwasserstoffen als organisch lipophile Verbindung (b) ist daher erfindungsgemäß.

Die Oxidationsmittelzubereitung enthält als organisch lipophile Verbindung (b) daher Paraffinkohlenwasserstoffe.

Es ist weiterhin besonders bevorzugt, wenn die Oxidationsmittelzubereitung als organisch lipophile Verbindung(en) (b) mindestens ein planzliches Fett, mindestens ein pflanzliches Öl und/oder Paraffinkohlenwasserstoffe enthält.

Des Weiteren ist es ebenfalls besonders bevorzugt, wenn die Oxidationsmittelzubereitung als organisch lipophile Verbindung(en) (b) mindestens ein pflanzliches Fett und/oder ein pflanzliches Öl und Paraffinkohlenwasserstoffe enthält.

Als organisch lipophile Verbindung(en) (b) können auch C₁₀-C₂₄-Fettalkohole eingesetzt werden.

Als C₁₀-C₂₄-Fettalkohole werden unverzeigte oder verzweigte, gesättigte, ein- oder mehrfach ungesättigte, aliphatische Alkohole mit 10 bis 24 C-Atomen bezeichnet. Beispiele für gesättigte C₁₀-C₂₄-Fettalkohole sind Decan-1-ol, Undecan-1-ol, Dodecan-1-ol (Dodecylalkohol, Laurylalkohol), Tridecan-1-ol. Tetradecan-1-ol (Tetradecylalkohol, Myristylalkohol), Pentadecan-1-ol, Hexadecan-1-ol (Hexadecylalkohol, Cetylalkohol, Palmitylalkohol), Heptadecan-1-ol, Octadecan-1-ol (Stearylalkohol). Nonadecan-1-ol, Eisosan-1-ol (Eicasylalkohol, Arachylalkohol), Heneicosan-1-ol und Docosan-1-ol (Docosylalkohol, Behenylalkohol).

Beispiele für ein- bzw. mehrfach ungesättigte C₁₀-C₂₄-Fettalkohole sind (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenylalkohol), (9*Z*)-Eicos-9-en-1-ol (Gadoleylalkohol) und (5*Z*,8*Z*,11*Z*,14*Z*)-Eicosa-5,8,11,14-tetraen-1-ol (Arachidonalkohol).

Weiterhin können Silikonöle als organisch lipophile Verbindung(en) (b) in den erfindungsgemäßen Oxidationsmittelzubereitungen eingesetzt werden. In die Gruppe der Silikonöle fallen vor allem lineare, polymere Polydimethylsiloxane, die nach dem Schema (R₂SiO)ₓ aufgebaut sind (mit R = Methyl) und molare Massen zwischen 1 000 und 150 000 g/mol aufweisen. Diese Öle sind in der Regel hydrophobe Flüssigkeiten.

Schließlich können auch Dialkylether der Formel (I) als organisch lipophile Verbindung(en) (b) Einsatz finden,

R1-O-R2 (I)

worin
R1 und R2 unabhängig voneinander für eine unverzeigte oder verzweigte C₈-C₂₄-Alkylgruppe stehen.

Die Dialkylether der Formel (I) können unsymmetrisch sein, sind vorzugsweise aber symmetrisch substituiert.

Exemplarisch zu nennende unverzeigter Dialkylether der Formel (I) sind der Di-n-octylether, im Handel erhältlich unter dem Namen Cetiol OE (INCI-Name: DICAPRYLYL ETHER) und der Di-n-stearylether. Ein Beispiel für Dialkylether der Formel (I) mit verzweigten C₈-C₂₄-Alkylgruppen ist der Di-2-ethylhexylether.

Die organisch lipophilen Verbindung(en) (b) können in einer Menge von 0,01 bis 15,0 Gew.-% in der erfindungsgemäßen Oxidationsmittelzubereitung enthalten sein, wobei die Oxidationsmittelzubereitung 5,0 bis 14,0 Gew.-% Paraffinkohlenwasserstoffe enthält. Hierbei bezieht sich der Gewichtsbereich von 0,01 bis 15 Gew.-% auf die Summe aller in der Oxidationsmittelzubereitung enthaltenen lipophilen Verbindungen (b). Bevorzugt sind die organisch lipophilen Verbindungen (b) in einer Menge von 0,5 bis 14,5 Gew.-%, weiter bevorzugt von 5,0 bis 14,0 Ges.-% und ganz besonders bevorzugt von 8,0 bis 13,5 Gew.-% in der Oxidationsmittelzubereitung enthalten.

Als dritten erfindungswesentlichen Formulierungsbestandteil (c) enthalten die Oxidationsmittelzubereitungen 0,01 bis 2,0 Gew.-% eines oder mehrerer organischer Verdicker aus der Gruppe der Polysaccharide, wobei die Oxidationsmittelzubereitung 0,1 bis 1,0 Gew.-% Xanthan Gum enthält.

Als organische Verdicker aus der Gruppe der Polysaccharide (c) können Vertreter der Cellulosen (Cellulose selbst sowie ihre Derivate), der Alginsäuren (sowie ihre entsprechenden physiologisch verträglichen Salze, die Alginate), Agar Agar (mit dem in Agar Agar als Hauptbestandteil vorhandenen Polysaccharid Agarose), Stärke-Fraktionen und Derivate wie Amylose, Amylopektin und Dextrine, Karaya-Gummi, Johannisbrotkernmehl, Gummi Arabicum, Dextrane, Guar Gum und Xanthan Gum eingesetzt werden.

Geeignete Cellulose-Derivate sind Methylcellulosen, Ethylcellulosen, Hydroxyalkylcellulosen (wie beispielsweise Hydroxyethylcellulose), Methylhydroxyalkylcellulosen und Carboxymethylcellulosen sowie ihre physiologisch verträglichen Salze.

Methylcellulosen werden beispielsweise unter den Namen Culminal von der Firma Hercules oder unter dem Namen Methocel von der Firma Dow vertrieben. Hydroxypropyl methylcellulosen sind zum Beispiel unter den Handelsnamen Methylcellulose MK 70 M und Methylcellulose MK 10 M von der Firma Konimpex kommerziell erhältlich.

Als physiologisch verträgliche Salze der Alginsäure können beispielsweise Natriumalginat, Ammoniumalginat, Calciumalginat und Magnesiumalginat eingesetzt werden.

Bevorzugt werden aus der Gruppe der Polysaccharide (c) für die Verdickung der erfindungsgemäßen Oxidationsmittelzubereitungen die anionischen Polysaccharide ausgewählt.

Als besonders vorteilhaft im Hinblick auf eine zuverlässige Viskositätseinstellung und rückstandsfreie Anwendung auf Keratinfasern und der Kopfhaut haben sich als anionische Polysaccharide die Carboxymethylcellulosen, die Alginsäuren, Xanthan Gum und/oder deren physiologisch verträglichen Salze erwiesen.

Die erfindungsgemäßen Oxidationsmittelzubereitungen enthalten als Polysaccharid (c) Xanthan Gum.

Carboxymethylcellulosen, Alginsäuren und Xanthan Gum werden neben ihren physiologisch verträglichen Salzen im Rahmen der vorliegenden Erfindung ebenfalls als anionische Polysaccharide bezeichnet, da die in diesen Polysacchariden vorhandenen Carbonsäuregruppierungen in Wasser bzw. wasserhaltiger Formulierung zwangsläufig in mehr oder weniger starkem Ausmaß dissoziieren, wodurch anionische Carboxylat-Gruppen gebildet werden, deren Anzahl mit steigendem pH-Wert weiter zunimmt. Da die Anwendung der Oxidationsfärbemittel bei alkalischen pH-Werten erfolgt, sind die vorgenannten Polysaccharide in Formulierung daher zwangsläufig anionisch.

Die Polysaccharide (c) sind in der Oxidationsmittelzubereitung in Mengen von 0,01 bis 2,0 Gew.-%, bevorzugt von 0,05 bis 1,5 Gew,-%, weiter bevorzugt von 0,1 bis 1,0 Gew.-% und ganz besonders bevorzugt von 0,15 bis 0,5 Gew.-% enthalten. Berechnungsgrundlage für die angegebenen Mengenbereiche ist die Summe aller in der Oxidationsmittelzubereitung enthaltenden Polysaccharide (c).

Die Oxidationsmittelzubereitung ist dadurch gekennzeichnet, dass sie als anionisches Polysaccharid 0,1 bis 1,0 Gew.-% und ganz besonders bevorzugt 0,15 bis 0,5 Gew.-% Xanthan Gum enthält.

Xanthan Gum wird beispielsweise unter dem Handelsnamen Keltrol von der Firma CP Kelco und unter den Namen Rhodicare, Rhodopol und Rhodigel von der Firma Rhodia vertrieben.

Die erfindungsgemäßen Oxidationsmittelzubereitungen können zusätzlich mindestens ein organisches Lösungsmittel enthalten, das ausgewählt ist aus 2-Butoxyethanol, Propylenglykol, Propylenglykolmonomethylether, Diethylengkykolmonomethylether, Diethylenglykolmonoethylether, Ethylenglykolmonobutylether, Ethylenglykolmonoethylether, Ethanol, Isopropanol, n-Propanol, n-Butanol, 1,2-Propandiol, 1,3-Propandiol, Glycerin, 1-Methoxy-2-propanol, 1-Ethoxy-2-propandiol, 1,4-Butandiol, 1,2-Hexandiol, Benzylalkohol, Phenoxyethanol, 2-Phenylethylalkohol, Ethylendigkykol, Methoxybutanol und n-Butylenglykol. Besonders bevorzugt ist es, wenn die erfindungsgemäße Oxidationsmittelzubereitung zusätzlich ein organisches Lösungsmittel enthält, das ausgewählt ist aus Propylenglykol und/oder Glycerin.

Die Lösungsmittel können in einer Menge von 0,1 bis 10,0 Gew.-%, bevorzugt von 1,0 bis 8,0 Gew.-%. weiter bevorzugt von 2,0 bis 7,0 Gew.-% und ganz besonders bevorzugt von 3,0 bis 6,0 Gew.-% in der Oxidationsmittelzubereitung vorhanden sein. Hierbei beziehen sich die angegebenen Mengenbereiche auf die Summe aller in der Oxidationsmittelformulierung enthaltenen Lösungsmittel aus der vorgenannten Gruppe.

In einer weiteren besonders bevorzugten Ausführungsform ist die erfindungsgemäße Oxidationsmittelzubereitung dadurch gekennzeichnet, dass sie zusätzlich 0,1 bis 10,0 Gew.-% mindestens eines organischen Lösungsmittels ausgewählt aus 2-Butoxyethanol, Propylenglykol, Propylenglykolmonomethylether, Diethylengkykolmonomethylether, Diethylenglykolmonoethylether, Ethylenglykolmonobutylether, Ethylenglykolmonoethylether, Ethanol, Isopropanol, n-Propanol, n-Butanol, 1,2-Propandiol, 1,3-Propandiol, Glycerin, 1-Mothoxy-2-propanol, 1-Ethoxy-2-propandiol, 1,4-Butandiol, 1,2-Hexandiol, Benzylalkohol, Phenoxyethanol, 2-Phenylethylalkohol, Ethylendigkykol, Methoxybutanol und n-Butylenglykol, besonders bevorzugt Propylenglykol und/oder Glycerin, enthält.

Zur Erfüllung der erfindungsgemäßen Aufgabenstellung hat es sich des Weiteren als vorteilhaft erwiesen, wenn die Oxidationsmittelzubereitungen zusätzlich mindestens ein organisches Carbonat enthalten. Als organische Carbonate werden die Diester der Kohlensäure bezeichnet, wobei die Kohlensäure mit zwei organischen Resten verestert ist. Hierbei kann die Kohlensäure mit zwei gleichen organischen Resten oder mit unterschiedlichen organischen Resten verestert sein. Auch zyklische Carbonate haben sich in diesem Zusammenhang als sehr gut geeignet erwiesen. Bei zyklischen Carbonaten handelt es sich bevorzugt um substituierte derviate des 1,3-Dioxolan-2-ons.

Es hat sich herausgestellt, dass besonders dann fein emulgierte bzw. dispergierte Emulsionen mit über einen langen Lagerzeitraum stabilen Viskositätswerten formuliert werden können, wenn die Oxidationsmittelzubereitungen zusätzlich mindestens ein organisches Carbonat enthalten, das ausgewählt ist aus Glycerincarbonat, Propylencarbonat, Ethylencarbonat, Dimethylcarbonat, Diethylcarbonat, Di-n-hexylcarbonat, Di-n-octylcarbonat, Di-n-decylcarbonat und Di-n-dodecylcarbonat.

Als vorteilhaft hat sich in diesem Zusammenhang vor allem erwiesen, eine Verbindung aus der Gruppe Di-n-hexylcarbonat, Di-n-octylcarbonat, Di-n-decylcarbonat und Di-n-dodecylcarbonat, ganz besonders bevorzugt Di-n-octylcarbonat, als zusätzlichen Bestandteil in die Oxidationsmittelzubereitung einzuarbeiten.

Die erfindungsgemäße Oxidationsmittelzubereitung ist dadurch gekennzeichnet, dass sie zusätzlich 0,1 bis 10,0 Gew.-% mindestens eines organischen Carbonats ausgewählt aus Glycerincarbonat, Propylencarbonat, Ethylencarbonat, Dimethylcarbonat, Diethylcarbonat, Di-n-hexylcarbonat, Di-n-octylcarbonat, Di-n-decylcarbonat und Di-n-dodecylcarbonat, bevorzugt Di-n-octylcarbonat, enthält.

Vorzugsweise werden die anwendungsbereiten Oxidationsmittelzubereitungen als flüssige Zubereitungen bereitgestellt und den Mitteln zusätzlich mindestens eine oberflächenaktive Substanz zugesetzt, wobei solche oberflächenaktive Substanzen je nach Anwendungsgebiet als Tenside oder als Emulgatoren bezeichnet werden. Prinzipiell können sie aus anionischen, kationischen, zwitterionischen, amphoteren und nichtionischen Tensiden und Emulgatoren ausgewählt werden.

Im Zuge der zu dieser Erfindung führenden Arbeiten hat sich jedoch gezeigt, dass die rheologischen Eigenschaften der Oxidationsmittelzubereitung insbesondere dann vorteilhaft beeinflusst werden, wenn die erfindungsgemäßen Mittel nichtionogene grenzflächenaktive Stoffe enthalten. Demnach ist es besonders bevorzugt, wenn die erfindungsgemäße Oxidationsmittelzubereitung zusätzlich mindestens ein nichtionisches Tensid enthält.

Als besonders geeignete nichtionische Tenside haben sich hierbei Alkylenoxid-Anlagerungsprodukte an Fettalkohole mit jeweils 2 bis 80 Mol Ethylenoxid pro Mol Fettalkohol erwiesen.

Eine weitere besonders bevorzugte Ausführungsform der vorliegenden Erfindung ist dadurch gekennzeichnet, dass die Oxidationsmittelzubereitung zusätzlich 0,1 bis 10 Gew.-% mindestens eines polyethoxylierten Fettalkohols der Formel (IV) enthält, 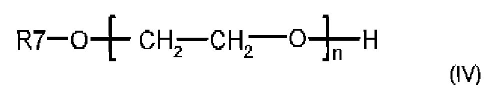 worin
R7 für eine unverzweigte oder verzweigte, gesättigte oder ungesättigte C₁₀-C₂₄-Alkylgruppe steht und
n für eine ganze Zahl von 2 bis 80 steht.

Bevorzugt steht R7 für eine unverzeigte, gesättigte C₁₂-C₁₈-Alkylgruppe oder für eine unverzeigte, einfach ungesättigte C₁₂-C₁₈-Alkylgruppe.

n steht bevorzugt für eine ganze Zahl von 10 bis 40, ganz besonders bevorzugt steht n für eine ganze Zahl von 10 bis 25.

Beispiele für alkoxylierte Fettalkohole der Formel (IV) sind Laureth-2, Laureth-3, Laureth-5, Laureth-8, Laureth-10, Laureth-12, Laureth-15, Laureth-20, Laureth-25, Laureth-30, Laureth-40, Laureth-50, Myreth-2, Myreth-3, Myreth-5, Myreth-8, Myreth-10, Myreth-12, Myreth-15, Myreth-20, Myreth-25, Myreth-30, Myreth-40, Myreth-50, Ceteth-2, Ceteth-3, Ceteth-5, Ceteth-8, Ceteth-10, Ceteth-12, Ceteth-15, Ceteth-20, Ceteth-25, Ceteth-30, Ceteth-40, Ceteth-50, Steareth-2, Steareth-3, Steareth-5, Steareth-8, Steareth-10, Steareth-12, Steareth-15, Steareth-20, Steareth-25, Steareth-30, Steareth-40, Steareth-50, Ceteareth-2, Ceteareth-3, Ceteareth-5, Ceteareth-8, Ceteareth-10, Ceteareth-12, Ceteareth-15, Ceteareth-20, Ceteareth-25, Ceteareth-30, Ceteareth-40, Ceteareth-50, Oleth-2, Oleth-3, Oleth-5, Oleth-8, Oleth-10, Oleth-12, Oleth-15, Oleth-20, Oleth-25, Oleth-30, Oleth-40 und Oleth-50.

Besonders bevorzugt werden Ceteth-12, Ceteth-20, Steareth-12, Steareth-20, Ceteareth-12, Ceteareth-20 und/oder deren Gemische als nichtionisches Tensid in den erfindungsgemäßen Oxidationsmittelzubereitungen eingesetzt.

Die polyethoxylierten Fettalkohole der Formel (IV) können in Mengen von 0,1 bis 10,0 Gew.-%, bevorzugt von 0,5 bis 7,5 Gew.-%, weiter bevorzugt von 1,0 bis 5,0 Gew.-% und ganz besonders bevorzugt von 1,5 bis 2,5 Gew.-% in der Oxidationsmittelzubereitung eingesetzt werden. Hierbei beziehen sich die angegebenen Mengenbereiche auf die Summe aller in der Oxidationsmittelzubereitung enthaltenen Verbindungen der Formel (IV).

Weiterhin können die erfindungsgemäßen Mittel auch nichtionische Tenside eines von den polyethoxylierten Fettalkoholen unterschiedlichen Typs sowie anionische, kationische, zwitterionische und amphotere Tenside beinhalten. Ebenfalls geeignete nichtionische Tenside sind Alkylpolyglykoside sowie Alkylenoxid-Anlagerungsprodukte an Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Weitere erfindungsgemäß geeignete Mittel sind dadurch gekennzeichnet, dass sie zusätzlich mindestens ein anionisches Tensid enthalten. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Ges.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäße Mittel sind auch dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Desweiteren können die erfindungsgemäßen Mittel dadurch gekennzeichnet sein, dass sie zusätzlich mindestens ein amphoteres Tensid enthalten. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Die von den polyethoxylierten Fettalkoholen der Formel (IV) verschiedenen Tenside werden in Anteilen von 0,1 bis 45 Gew.%, bevorzugt 1 bis 30 Gew.% und ganz besonders bevorzugt von 1 bis 15 Gew.% eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid^{®} S 18 im Handel erhältliche Stearamidopropyl-dimethylamin dar, Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Zur Erzielung einer verstärkten Aufhell- und Bleichwirkung kann die Oxidationsmittelzubereitung weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-% in dem erfindungsgemäßen Mittel enthalten.

Die Oxidationsmittelzubereitungen können weiterhin zusätzliche Wirk-, Hilfs- und Zusatzstoffe enthalten, um die Aufhellleistung zu verbessern und weitere gewünschte Eigenschaften der Mittel einzustellen. So kann es sich als vorteilhaft erweisen, wenn die Mittel mindestens ein biologisch unbedenkliches Verdickungsmittel enthalten, welche von den Mitteln des ersten Erfindungsgegenstandes verschieden sind. Geeignet sind hierbei insbesondere anorganische Verdickungsmittel, insbesondere Schichtsilikate wie beispielsweise Bentonit, besonders Smektite, wie Montmorillonit oder Hectorit.

Die erfindungsgemäße Oxidationsmittelzubereitung ist zur Stabilisierung des in ihr enthaltenen Wasserstoffperoxids üblicherweise auf einen sauren pH-Wert eingestellt. Als Säuren können Mineralsäuren (wie beispielsweise Salzsäure, Schwefelsäure oder Phosphorsäure) oder Genußsäuren (wie beispielsweise Zitronensäure, Weinsäure oder Äpfelsäure) eingesetzt werden. Der gewünschte pH-Wert kann auch durch weitere organische Säuren aus der Gruppe der Phosphonsäure und/oder Diphosphonsäuren, wie beispielsweise Etidronsäure (1-Hydroxyethan-1,1-diphosphonsäure), oder aus der Gruppe der heterozyklischen Säuren, wie beispielsweise 2,6-Dipicolinsäure (2,6-Dicarboxypyridin), eingestellt werden.

Die vom Konsumenten bzw vom Friseur angewendeten Produkte zum Färben bzw. Aufhellen/Bleichen von Keratinfasern bestehen aus mindestens zwei getrennt voneinander konfektionierten Komponenten.

Die erfindungsgemäße Oxidationsmittelzubereitung stellt hierbei die erste Komponente dar, welche kurz vor der Anwendung mit einer alkalisch eingestellten zweiten Komponente vermischt wird. Diese zweite Komponente liegt meist in Form einer kosmetischen Cremeformulierung vor und enthält im Fall eines oxidativen Färbeproduktes auch Oxidationsfarbstoffvorprodukte und gegebenenfalls zusätzlich direktziehende Farbstoffe.

Wird das Produkt als oxidatives Färbemittel eingesetzt, so enthält die alkalisch eingestellte zweite Komponente mindestens ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-y)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-ethylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphanyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethy[)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-hydroxyindol, 4-Hydroxyindolin, 6-Hydroxyindolin, 7-Hydroxyindolin oder Gemischen dieser Verbindungen oder deren physiologisch verträglichen Salzen.

Zusätzlich kann die alkalisch eingestellte zweite Komponente neben Oxidationsfarbstoffvorprodukten auch direktziehende Farbstoffe enthalten. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe,

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropy])amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethyl-amino-3-nitrobenzoesäure und 2-Chlor-6-ethylamino-4-nitrophenol.

Die Entwicklerkomponenten, Kupplerkomponenten und gegebenenfalls direktziehenden Farbstoffe werden bevorzugt jeweils in einer Menge von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Aufhell- und Färbeprozesse auf Keratinfasern laufen üblicherweise im alkalischen Milieu ab. Um die Keratinfasern und auch die Haut soweit wie möglich zu schonen, ist die Einstellung eines zu hohen pH-Wertes jedoch nicht wünschenswert. Der pH-Wert der anwendungsbereiten Mittel kann daher zwischen 3 und 11 liegen. Es ist jedoch bevorzugt, wenn der pH-Wert des anwendungsbereiten Mittels zwischen 7 und 11, insbesondere zwischen 8,5 und 10,5 liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.

In einer weiteren bevorzugten Ausführungsform kann die Wirkung des anwendungsbereiten Mittels durch Emulgatoren gesteigert werden. Solche Emulgatoren sind beispielsweise
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®} 68,
- Anlagerungsprodukte von 5 bis 60 Mol Ethylenoxid an Rizinusöl und gehärtetes Rizinusöl,
- Phospholipide, vor allem Glucose-Phospolipide, die z. B. als Lecithine bzw. Phosphatidylcholine aus z.B. Eidotter oder Pflanzensamen (z.B. Sojabohnen) gewonnen werden,
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls^{®} PGPH)
- Lineare und verzweigte Fettsäuren mit 8 bis 30 C-Atomen und deren Na-, K-, Ammonium-, Ca-, Mg- und Zn-Salze.

Die erfindungsgemäßen Mittel enthalten die Emulgatoren bevorzugt in Mengen von 0,1 bis 25 Gew.-%, insbesondere 0,5 bis 15 Gew.-%.

Nichtionogene Emulgatoren bzw. Tenside mit einem HLB-Wert von 10-15 können erfindungsgemäß besonders bevorzugt sein. Unter den genannten Emulgatoren-Typen können die Emulgatoren, welche kein Ethylenoxid und/oder Propylenoxid im Molekül enthalten ganz besonders bevorzugt sein.

Ferner können die Oxidationsmittelzubereitung und/oder die alkalisch eingestellte zweite Komponente weitere Wirk-, Hilfs- und Zusatzstoffe enthalten. Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-% eingesetzt.

Die anwendungsbereite Formulierung zur Farbveränderung der Keratinfasern wird kurz vor der Anwendung durch Vermischen der Oxidationsmittelzubereitung mit einer zweiten alkalischen Komponente hergestellt. Die erfindungsgemäße Oxidatiansmittelzubereitung lässt sich insbesondere dann ausgezeichnet mit der zweiten Komponente vermischen, wenn für den Mischvorgang eine Anmischschale verwendet wird. Um beide Komponenten mit einem Pinsel bzw, einer Applicette vollständig und in kurzer Zeit homogen miteinander zu verrühren, ist es maßgeblich, dass die Viskosität der Oxidationsmittelzubereitung innerhalb eines bestimmten Bereiches liegt.

Im Sinne dieser Erfindung geeignete Oxidationsmittelzubereitungen zeichnen sich dadurch aus, dass sie bei 22 °C eine Viskosität von 70 bis 900 mPas, bevorzugt von 100 bis 700 mPas, weiter bevorzugt von 150 bis 500 mPas und ganz besonders bevorzugt von 180 bis 300 mPas besitzen. Alle angegebenen Viskositätswerte beziehen sich hierbei auf Werte, die mit einem Brookfield-Viskosimeter (Rotationsviskosimeter) bei 22 °C mit Spindel 5 bei 4 Umdrehungen pro Minute (Upm) gemessen wurden.

Eine weitere besonders bevorzugte Ausführungsform der vorliegenden Erfindung ist dadurch gekennzeichnet, dass die Oxidationsmittelzubereitung eine Viskosität von 70 bis 900 mPas (22 °C / Brookfield-Viskosimeter / Spindel 5 / 4 Upm) besitzt.

In handelsüblichen Färbe- bzw. Blondierprodukten werden die Oxidationsmittelzubereitung und die zweite Komponente meist getrennt konfektioniert in zwei Flaschen bzw. Behältnissen angeboten. Für die Herstellung der Anwendungsformulierung muß der Inhalt beider Behältnisse möglichst vollständig in eine Anmischschale überführt werden. Zur Gewährleistung einer nach Möglichkeit kompletten Überführung aus dem Behältnis in die Anmischschale ist die Einstellung der richtigen Viskosität ebenfalls von entscheidender Bedeutung. Es ist insbesondere von Bedeutung, dass die gesamte Menge der vorhandenen Oxidationsmittelzubereitung für den Farbveränderungsprozeß zur Verfügung gestellt wird.

Als quantitatives Maß für die Entnehmbarkeit der Oxidationsmittelzubereitung aus ihrem Behältnis kann die Ausbringrate (Angabe in %) herangezogen werden. Die Ausbringrate gibt das Gewichtsverhältnis der aus dem Behältnis entnehmbaren Oxidationsmittelzubereitung zu der im Behältnis vorhandenen Gesamtmenge an. Je höher die Ausbringrate ist, desto größer ist der Anteil der Oxidationsmittelzubereitung, der für den Färbe- bzw. Blondierprozeß zur Verfügung steht. Ausbringraten kleiner 90 % sind aus ökonomischen und ökologischen Gesichtspunkten nicht akzeptabel.

Auch die Viskosität der finalen Anwendungsmischung wird entscheidend von den in der Oxidationsmittelzubereitung enthaltenen Verdickern bestimmt. Hierbei nehmen sowohl die Art der eingesetzten Verdicker, ihre Einsatzmengen, ihre jeweiligen Abhängigkeiten von Temperatur und pH-Wert sowie zusätzliche Wechselwirkungen, die bei Einsatz mehrere Verdicker untereinander auftreten können, Einfluß auf die Viskosität der Anwendungsmischung. Unter Berücksichtigung dieser Einflußfaktoren muß die Viskosität der Anwendungsformulierung so eingestellt sein, dass die Anwendungsformulierung noch dünnflüssig genug ist, um die Keratinfasern optimal zu benetzen und eine ausreichend schnelle Diffusion von Oxidationsmittel bzw. Farbstoffvorpodukten in die Keratinfasern hinein zu gewährleisten, gleichzeitig aber nicht so dünnflüssig ist, dass die Anwendungsformulierung von den Keratinfasern hinunter tropft.

Es hat sich herausgestellt, dass Anwendungsformulierungen, deren Viskosität innerhalb eines Bereiches von 22 000 bis 30 000 mPas liegen, alle Anforderungen in optimaler Weise erfüllen.

Bevorzugte Oxidationsmittelzubereitungen sind daher weiterhin dadurch gekennzeichnet, dass sie die erfindungswesentlichen Bestandteile in solchen Mengen enthalten, dass die Viskosität der nach dem Mischvorgang mit einer standardisierten alkalischen Komponente erhaltenen Anwendungsformulierung innerhalb eines Bereiches von 22 000 bis 30 000 mPas, bevorzugt von 23 000 bis 29 000 mPas und besonders bevorzugt von 24 000 bis 28 000 mPas (22 °C / Brookfield-Viskosimeter / Spindel 5 / 4 Upm) liegt.

Als standardisierte alkalische Komponente wird in diesem Zusammenhang eine handelsübliche Creme verstanden, welche die nachfolgenden, das Fließverhalten beeinflussenden Bestandteile enthält:
Cetearylalkohol (8,25 Gew.-%), Lorol techn. (C12-C18 Fettalkohole) (2,75 Gew.-%), Ceteareth-20 (3,0 Gew.-%), Dehyquart A CA (INCI: cetrimonium chloride), 24-26 %ig (3,0 Gew.-%). Ammoniak (25 %ige, wässrige Lsg.) (6,9 Gew.-%)

Zur Anwendung der erfindungsgemäßen Oxidationsmittelzubereitungen eignet sich insbesondere ein Verfahren zur Farberänderung von keratinischen Fasern, insbesondere menschlichen Haaren, welches dadurch gekennzeichnet ist, dass die Oxidationsmittelzubereitung des ersten Erfindungsgegenstandes mit einem weiteren, getrennt konfektionierten Mittel, enthaltend ein Alkalisierungsmittel und gegebenenfalls zusätzlich Oxidationsfarbstoffvorpodukte und/oder direktziehende Farbstoffe, vermischt wird, auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt erfolgt die Vermischung der Oxidationsmittelformulierung mit der zweiten Komponente in einer Anmischschale, wobei das Vermischen durch Verrühren mit einem Pinsel bzw. mit einer Aplizette erfolgt. Bevorzugt beträgt die Einwirkzeit der anwendungsbereiten Färbemittel 5 bis 45 min, insbesondere 10 bis 40 min, besonders bevorzugt 15 bis 35 min, Während der Einwirkzeit des Mittels auf der Faser kann es vorteilhaft sein, den Aufhellvorgang durch Wärmezufuhr zu unterstützen. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhell- bzw. Färbemittel einen stark tensidhaltigen Träger beisitzt.

Ein weiterer Gegenstand der vorliegenden Erfindung ist ein Verfahren zur Farbveränderung von Haaren, welches dadurch gekennzeichnet ist, dass
(I) eine Oxidationsmittelzubereitung des ersten Erfindungsgegenstands zusammen mit
(II) einem Mittel enthaltend ein Alkalisierungsmittel und gegebenenfalls zusätzlich mindestens ein Oxidationsfarbstoffvorprodukt vom Entwicklertyp und/oder Kupplertyp und/oder einen direktziehenden Farbstoff
(III) in einer Anmischschale durch Rühren mit einem Pinsel oder einer Applicette vollständig vermischt wird,
(IV) die auf diese Weise hergestellte homogene Anwendungsmischung mit einem Pinsel oder einer Aplizette auf die Haare aufgebracht wird,
(V) dort für einen Zeitraum von 5 bis 60 Minuten belassen und
(VI) anschließend wieder mit Wasser oder einem Shampoo ausgespült wird.

Vor der Anwendung liegen die Oxidationsmittelzubereitung und die das Alkalisierungsmittel enthaltende Komponente getrennt voneinander konfektioniert vor, Weiterhin kann die das Alkalisierugsmittel enthaltende Komponente zusätzlich einen oder mehrere der zuvor genannten Formulierungsbestandteile enthalten.

In einer weiteren Ausführungsform kann die anwendungsbereite Formulierung durch Vermischen von 3 verschiedenen, getrennt voneinander konfektionierten Komponenten erhalten werden. Hierbei handelt es sich bei der ersten Komponente um die Oxidationsmittelzubereitung des ersten Erfindungsgegenstandes und bei der zweiten Komponente um eine alkalisch eingestellte Formulierung, welche beide mit einer weiteren, dritten Komponente vermischt werden. Bei dieser dritten Komponente kann es sich sowohl um eine flüssige Komponente handeln, die in Form eines Gels, einer Emulsion, einer Dispersion oder einer Lösung vorliegt. Es ist aber ebenfalls erfindungsgemäß, wenn diese dritte Komponente als Feststoff in Form eines Pulvers oder einer Tablette zu den anderen beiden Komponenten hinzugefügt und durch Rühren mit diesen vermischt wird.

In einer weiteren bevorzugten Ausführungsform des erfindungsgemäßen Verfahrens werden daher
(I) eine Oxidationsmittelzubereitung des ersten Erfindungsgegenstands zusammen mit
(II) einem Mittel enthaltend ein Alkalisierungsmittel und gegebenenfalls zusätzlich mindestens ein Oxidationsfarbstoffvorprodukt vom Entwicklertyp und/oder Kupplertyp und/oder einen direktziehenden Farbstoff
(III) und einer dritten, flüssigen oder festen, Komponente
(IV) in einer Anmischschale durch Rühren mit einem Pinsel oder einer Applicette vollständig vermischt,
(V) die auf diese Weise hergestellte homogene Anwendungsmischung mit einem Pinsel oder einer Aplizette auf die Haare aufgebracht,
(VI) dort für einen Zeitraum von 5 bis 60 Minuten belassen und
(VII) anschließend wieder mit Wasser oder einem Shampoo ausgespült.

Diese dritte Komponente kann einen oder mehrere der zuvor genanten Formulierungsbestandteile enthalten. Bevorzugt enthält die dritte Komponente Peroxo-Salze zur Verstärkung der Blondier- bzw. Aufhellwirkung oder Strukturanten, haarkonditionierende Verbindungen, faserstrukturverbessernde Wirkstoffe, Vitamine, Provitamine und/oder Vitaminvorstufen.

Im Zuge der zu dieser Erfindung führenden Arbeiten hat sich herausgestellt, dass bereits kleine Mengen organischer Verdicker aus der Gruppe der Polysaccharide (c) in der Lage sind, die Viskosität von Oxidationsmittelzubereitungen enthaltend Wasserstoffperoxid (a) und organisch lipophile Verbindung(en) (b) genau, zuverlässig und stabil auf einen gewünschten Spezifikationsbereich einzustellen.

Ein weiterer Gegenstand der vorliegenden Erfindung ist die Verwendung von organischen Verdickern aus der Gruppe der Polysaccharide (c) in einem Mengenbereich von 0,01 bis 2,0 Gew.-% zur Viskositätseinstellung von Oxidationsmittelzubereitungen, die
(a) 0,01 bis 25,0 Gew.-% Wasserstoffperoxid und
(b) 0,01 bis 15,0 Gew.-% einer oder mehrerer organisch lipophiler Verbindung(en) aus der Gruppe der pflanzlichen und tierischen Fette, Öle und Wachse, der Paraffinkohlenwasserstoffe, der C₁₀-C₂₄-Fettalkohole, der Silikonöle und der Dialkylether der Formel (I),

   R1-O-R2 (I)

   worin
   R1 und R2 unabhängig voneinander für eine unverzeigte oder verzweigte C₈-C₂₄-Alkylgruppe stehen, enthalten.

Bezüglich weiterer bevorzugter Ausführungsformen der erfindungsgemäßen Verfahren und Verwendung gilt mutatis mutandis das zu den erfindungsgemäßen Mitteln Gesagte.

### Ausführungsbeispiele

Es wurden die folgenden Rezepturen hergestellt. Die Mengenangaben verstehen sich, sofern nichts anderes vermerkt ist, jeweils in Gewichtsprozent.

### 1.1 Oxidationsmittelzubereitungen

| Formulierungsbestandteile (Vergleich) | V1 (Gew.-%) | V2 (Gew.-%) |
|---|---|---|
| Natriumlaurylethersulfat (INCI: Sodium Laureth Sulfate) | 2.00 | --- |
| Cetylalkohol | --- | 3,5 |
| Ceteareth-20 | --- | 0,5 |
| Ceteareth-30 | --- | 1,5 |
| Natriumhydroxid, 45 %ig, techn. | 0,73 | --- |
| Kaliumhydroxid, 50 %ig | --- | 0,15 |
| Dipicolinsäure | 0,1 | 0,1 |
| Dinatriumpyrophosphat | 0,03 | 0,03 |
| HEDP, 60 %ig (INCI: Etidronic Acid) | 1,5 | 0,08 |
| Dow Corning DB 110 A (INCI: Dimethicone) | 0,07 | --- |
| Aculyn 33 A (INCI: Acrylates Copolymer), 27-29% | 10,0 | 10,0 |
| Wasserstoffperoxid, 50 %ige Lsg. | 22,4 | 12,0 |
| Wasser | ad 100 | ad 100 |

| Formulierungsbestandteil (erfindungsgemäß) | E1 (Gew.-%) | E2 (Gew.-%) | E3 Gew.-%) |
|---|---|---|---|
| Emulgade SE-PF | 4,5 | 4,5 | 4,5 |
| CETEARETH-12 | 2,0 | 2,0 | 2,0 |
| Myritol 331 | 3,0 | 3,0 | 3,0 |
| Cetiol CC (INCI: Dicaprylyl Carbonate) | 5.0 | 5,0 | 5,0 |
| Glycerin (96 %ig) | 5,0 | 5,0 | 5,0 |
| Paraffinum Liquidum | 10,0 | 10,0 | 10,0 |
| Dipicolinsäure | 0,1 | 0,1 | 0.1 |
| Dinatriumpyrophosphat | 0,1 | 0,1 | 0,1 |
| HEDP, 60 %ig (INCI: Editronic Acid) | 0,06 | 0,06 | 0,06 |
| Kaliumhydroxid (50 %ig) | 0,12 | 0,12 | 0,12 |
| Keltrol CG-SFT (INCI: Xanthan Gum) | 0,50 | 0,20 | 0,10 |
| Propylenglykol | 1,0 | 0,4 | 0.2 |
| Wasserstoffperoxid, 50 %ige Lsg. | 23,0 | 23,0 | 23,0 |
| Wasser (dest.) | ad 100 | ad 100 | ad 100 |

### Formulierungsbestandteile

| | |
|---|---|
| Emulgade SE-PF: | INCI: glcyeryl stearate, ceteareth-20, ceteareth-12, cetearyl alcohol, cetyl palmitate |
| Myritol 331: | Glycerides, mixed coco, decanoyl and octanoyl (INGI: cocogylcerices) |

### 1.2 Färbecreme

Die Oxidationsmittelzubereitungen wurden jeweils im Verhältnis 1:1 mit der folgenden Färbecreme in einer Anmischschale vermischt und unter Verwendung einer Applicette durchgerührt. Hieraus resultierten die Anwendungsmischungen.

### Färbecreme FC

| Formulierungsbestandteil | Gew.-% |
|---|---|
| Cetearylalkohol | 8,25 |
| Lorol techn. (C₁₂-C₁₈Fettalkohole) | 2,75 |
| Ceteareth-20 | 3,0 |
| Dehyquart A CA (INCI: Cetrimonium Chloride), 24-26 %ig | 3,0 |
| Natriumsulfit | 0,5 |
| Ammoniumsulfat | 0,5 |
| p-Toluylendiamin, Sulfat | 0,7 |
| Resorcin | 0,17 |
| 4-Chlorresorcin | 0,30 |
| 4-Amino-3-methylphenol | 0,14 |
| p-Amino-o-cresol (5-Amino-2-methylphenol) | 0,1 |
| HEDP, 60 %ig (INCI: Etidronic Acid) | 0,2 |
| Ammoniak (25 %ige, wässrige Lsg.) | 6,9 |
| Ascorbinsäure | 0,5 |
| Propylenglykol | 0,31 |
| Natriumsilikat 42 | 0,5 |
| Gluadin W 40 (INCI: Hydrolyzed Wheat Protein) | 0,2 |
| Wasser (dest.) | ad 100 |

### 1.3 Bestimmung von Viskositäten und Ausbringraten

Mit einem Brookfield-Viskosimeter (Rotationsviskosimeter) wurden bei 22 °C (Spindel 5 / 4 Umdrehungen pro Minute) die Viskositäten der Oxidationsmittelzubereitungen und der Anwendungsmischungen bestimmt. Zusätzlich wurden alle Formulierungen von einer fachkundigen Person im Hinblick auf ihre rheologischen Eigenschaften objektiv bewertet. Zusätzlich wurde das ökologische Profil des als Verdicker eingesetzten Polymers bewertet.

### Eigenviskosität der Oxidationsmittelzubereitungen

| | V1 | V2 | E1 | E2 | E3 |
|---|---|---|---|---|---|
| Viskosität (22 °C, Brookfield, Spindel 5 / 4 Upm) [mPas] | 30 | 5840 | 840 | 210 | 80 |
| Mischbarkeit mit Färbecreme | nicht mischbar | nicht mischbar | gut mischbar | gut mischbar | gut mischbar |
| Biopolymer als Verdicker | nein | nein | ja | ja | ja |

Zur Bewertung der Überführbarkeit der Oxidationsmittelzubereitung aus dem Lagerbehältnis in die Anmischschale wurden die Ausbringraten bestimmt. Hierzu wurden die Behältnisse zunächst mit einer definierten Menge der Oxidationsmittelzubereitung gefüllt und diese dann für 24 Stunden stehen gelassen. Anschließend wurden die Behältnisse möglichst vollständig geleert. Durch Differenzwiegung wurde das Gewicht der aus dem Behältnis entnehmbaren Oxidationsmittelzubereitung bestimmt. Die Ausbringrate ergibt sich jeweils als das Verhältnis des Gewichtes der entnommenen Formulierung zur in der im Behältnis befindlichen Gesamtmenge.

### Ausbringraten

| | V1 | V2 | E1 | E2 | E3 |
|---|---|---|---|---|---|
| Ausbringrate | 98 % | 63,5 % | 90 % | 93 % | 93 % |
| | akzeptabel | nicht akzeptabel | akzeptabel | akzeptabel | akzeptabel |

### Viskosität der Anwendungsmischung

| | V1 + FC | V2 + FC | E1 + FC | E2 + FC | E3 + FC |
|---|---|---|---|---|---|
| Viskosität (22 °C, Brookfield, Spindel 5 / 4 Upm) [mPas] | 30 500 | 31 500 | 24 500 | 24 200 | 25 100 |

Ein Vergleich aller Ergebnisse zeigt, dass nur bei Anwendung der erfindungsgemäßen Oxidationsmittelzubereitungen alle Anforderungen im Hinblick auf gute Mischbarkeit und hohe Ausbringraten erfüllt werden können.

Die Anwendungsmischungen, die mit den erfindungsgemäßen Oxidationsmittelzubereitungen hergestellt wurden, zeigen ein im Hinblick auf Anwendungskomfort und Farbergebnis optimierten Viskositätsbereich.

## Patentansprüche

1. Oxidationsmittelzubereitung zur Behandlung von keratinischen Fasern, insbesondere von menschlichen Haaren, enthaltend in einem kosmetischen Träger
(a) 0,01 bis 25,0 Gew.-% Wasserstoffperoxid,
(b) 0,01 bis 15,0 Gew.-% einer oder mehrerer organisch lipophiler Verbindung(en) aus der Gruppe der pflanzlichen und tierischen Fette, Öle und Wachse, der Paraffinkohlenwasserstoffe, der C₁₀-C₂₄-Fettalkohole, der Silikonöle und der Dialkylether der Formel (I),
R1-O-R² (I)
worin
R1 und R2 unabhängig voneinander für eine unverzweigte oder verzweigte C₈-C₂₄-Alkylgruppe stehen,
wobei die Oxidationsmittelzubereitung 5,0 bis 14,0 Gew.-% Paraffinkohlenwasserstoffe enthält, und
(c) 0,01 bis 2,00 Ges.-% eines oder mehrerer organischer Verdicker aus der Gruppe der Polysaccharide,
wobei die Oxidationsmittelzubereitung 0,1 bis 1,0 Gew.-% Xanthan Gum enthält,
und
(d) 0,1 bis 10,0 Gew.-% mindestens eines organischen Carbonats ausgewählt aus Glycerincarbonat, Propylencarbonat, Ethylencarbonat, Dimethylcarbonat, Diethylcarbonat, Di-n-hexylcarbonat, Di-n-octylcarbonat, Di-n-decylcarbonat und Di-n-dodecylcarbonat,
mit der Maßgabe, dass die Oxidationsmittelzubereitung
- kein Polymer enthält, das durch Polymerisation oder Copolymerisation von Acrylsäure erhalten wird,
- kein Polymer enthält, das durch Polymerisation oder Copolymerisation von Methacrylsäure erhalten wird,
- kein Polymer enthält, das durch Polymerisation oder Copolymerisation von Acrylsäuerestern erhalten wird,
- kein Polymer enthält, das durch Polymerisation oder Copolymerisation von Methacrylsäureestern erhalten wird.

2. Oxidationsmittelzubereitung nach Anspruch 1, **dadurch gekennzeichnet, dass** sie als anionisches Polysaccharide (c) 0,15 bis 0,5 Ges.-% Xanthan Gum enthält.

3. Oxidationsmittelzubereitung nach einem der Ansprüche 1 bis 2, **dadurch gekennzeichnet, dass** sie zusätzlich 0,1 bis 10,0 Gew.-% mindestens eines organischen Lösungsmittels ausgewählt aus 2-Butoxyethanol, Propylenglykol, Propylenglykolmonomethylether, Diethylengkykolmonomethylether, Diethylenglykolmonoethylether, Ethylenglykolmonobutylether, Ethylenglykolmonoethylether, Ethanol, Isopropanol, n-Propanol, n-Butanol, 1,2-Propandiol, 1,3-Propandiol, Glycerin, 1-Methoxy-2-propanol, 1-Ethoxy-2-propandiol, 1,4-Butandiol, 1,2-Hexandiol, Benzylalkohol, Phenoxyethanol, 2-Phenylethylalkohol, Ethylendigkykol, Methoxybutanol und n-Butylenglykol, besonders bevorzugt Propylenglykol und/oder Glycerin, enthält.

4. Oxidationsmittelzubereitung nach einem der Ansprüche 1 bis 3, **dadurch gekennzeichnet, dass** sie zusätzlich 0,1 bis 10,0 Gew.-% Di-n-octylcarbonat enthält.

5. Oxidationsmittelzubereitung nach einem der Ansprüche 1 bis 4, **dadurch gekennzeichnet, dass** sie zusätzlich 0,1 bis 10 Gew.-% mindestens eines polyethoxylierten Fettalkohols der Formel (IV) enthält, 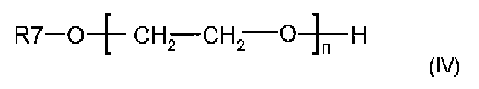 worin
R7 für eine unverzweigte oder verzweigte, gesattigte oder ungesattigte C₁₀-C₂₄-Alkylgruppe steht und
n für eine ganze Zahl von 2 bis 80 steht.

6. Oxidationsmittelzubereitung nach einem der Ansprüche 1 bis 5, **dadurch gekennzeichnet, dass** sie eine Viskosität von 70 bis 900 mPas (22 °C / Brookfield-Viskosimeter / Spindel 5 / 4 Upm) besitzt.

7. Oxidationsmittelzubereitung nach einem der Ansprüche 1 bis 6, **dadurch gekennzeichnet, dass** sie
(a) 0,01 bis 25,0 Gew.-% Wasserstoffperoxid,
(b) 8,0 bis 13,5 Gew.-% Paraffinkohlenwasserstoffe und
(c) 0,1 bis 1,0 Gew.-% Xanthan Gum enthalt.

8. Verfahren zur Farbveränderung von Haaren, **dadurch gekennzeichnet, dass**
(I) eine Oxidationsmittelzubereitung nach einem der Ansprüche 1 bis 7 zusammen mit
(II) einem Mittel enthaltend ein Alkalisierungsmittel und gegebenenfalls mindestens zusatzlich ein Oxidationsfarbstoffvorprodukt vom Entwicklertyp und/oder Kupplertyp und/oder einen direktziehenden Farbstoff
(III) in einer Anmischschale durch Rühren mit einem Pinsel oder einer Applicette vollständig vermischt wird,
(IV) die auf diese Weise hergestellte homogene Anwendungsmischung mit einem Pinsel oder einer Applicette auf die Haare aufgebracht wird,
(V) dort für einen Zeitraum von 5 bis 60 Minuten belassen und
(VI) anschließend wieder mit Wasser oder einem Shampoo ausgespült wird.

## Claims

1. An oxidizing agent preparation for treating keratinic fibers, in particular human hair, containing, in a cosmetic carrier
(a) from 0.01 to 25.0 wt.% hydrogen peroxide,
(b) from 0.01 to 15.0 wt.% of one or more organic lipophilic compound(s) from the group of plant and animal fats, oils and waxes, paraffin hydrocarbons, C₁₀-C₂₄ fatty alcohols, silicone oils and dialkyl ethers of formula (I),
R1-O-R2 (I)
in which
R1 and R2, independently of one another, represent an unbranched or branched C₈-C₂₄ alkyl group,
wherein the oxidizing agent preparation contains from 5.0 to 14.0 wt.% paraffin hydrocarbons,
and
(c) from 0.01 to 2.00 wt.% of one or more organic thickeners from the group of the
polysaccharides,
wherein the oxidizing agent preparation contains from 0.1 to 1.0 wt.% xanthan gum,
and
(d) from 0.1 to 10.0 wt.% of at least one organic carbonate selected from glycerol carbonate, propylene carbonate, ethylene carbonate, dimethyl carbonate, diethyl carbonate, di-n-hexyl carbonate, di-n-octyl carbonate, di-n-decyl carbonate and di-n-dodecyl carbonate,
with the proviso that the oxidizing agent preparation
- does not contain any polymer that is obtained by polymerization or copolymerization of acrylic acid,
- does not contain any polymer that is obtained by polymerization or copolymerization of methacrylic acid,
- does not contain any polymer that is obtained by polymerization or copolymerization of acrylic acid esters,
- does not contain any polymer that is obtained by polymerization or copolymerization of methacrylic acid esters.

2. The oxidizing agent preparation according to claim 1, **characterized in that** it contains from 0.15 to 0.5 wt.% xanthan gum as the anionic polysaccharide (c).

3. The oxidizing agent preparation according to either claim 1 or claim 2, **characterized in that** it additionally contains from 0.1 to 10.0 wt.% of at least one organic solvent selected from 2-butoxyethanol, propylene glycol, propylene glycol monomethyl ether, diethylene glycol monomethyl ether, diethylene glycol monoethyl ether, ethylene glycol monobutyl ether, ethylene glycol monoethyl ether, ethanol, isopropanol, n-propanol, n-butanol, 1,2-propanediol, 1,3-propanediol, glycerol, 1-methoxy-2-propanol, 1-ethoxy-2-propanediol, 1,4-butanediol, 1,2-hexanediol, benzyl alcohol, phenoxyethanol, 2-phenylethyl alcohol, ethylene diglycol, methoxybutanol and n-butylene glycol, particularly preferably propylene glycol and/or glycerol.

4. The oxidizing agent preparation according to one of claims 1 to 3, **characterized in that** it additionally contains from 0.1 to 10.0 wt.% di-n-octyl carbonate.

5. The oxidizing agent preparation according to one of claims 1 to 4, **characterized in that** it additionally contains from 0.1 to 10 wt.% of at least one polyethoxylated fatty alcohol of formula (IV), 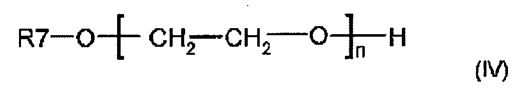 in which
R7 represents an unbranched or branched, saturated or unsaturated C₁₀-C₂₄ alkyl group and
n represents a whole number from 2 to 80.

6. The oxidizing agent preparation according to one of claims 1 to 5, **characterized in that** it has a viscosity of from 70 to 900 mPas (22 °C / Brookfield viscometer / spindle 5 / 4 rpm).

7. The oxidizing agent preparation according to one of claims 1 to 6, **characterized in that** it contains
(a) from 0.01 to 25.0 wt.% hydrogen peroxide,
(b) from 8.0 to 13.5 wt.% paraffin hydrocarbons, and
(c) from 0.1 to 1.0 wt.% xanthan gum.

8. A method for changing the color of hair, **characterized in that**
(I) an oxidizing agent preparation according to one of claims 1 to 7 and
(II) an agent containing an alkalizing agent and optionally in addition at least one oxidation dye precursor of the developer type and/or coupler type and/or a substantive dye
(III) are completely mixed together in a mixing bowl by stirring using a brush or an applicette,
(IV) the homogenous application mixture produced in this manner is applied to the hair using a brush or an applicette,
(V) is left there for a period of from 5 to 60 minutes and
(VI) is then rinsed out again using water or a shampoo.

## Revendications

1. Préparation d'agent oxydant pour le traitement de la fibre de kératine et en particulier des cheveux humains contenant dans un support cosmétique :
(a) de 0,01 à 25,0 % en poids de peroxyde d'hydrogène,
(b) de 0,01 à 15,0 % en poids de un ou plusieurs composés lipophiles organiques de groupe de graisses, huiles ou cires végétales ou animales, d'hydrocarbures paraffiniques, des alcools gras C₁₀-C₂₄, d'huiles de silicone et de dialkyléther de formule (I),
R1-O-R2 (I)
dans lequel
R1 et R2 représentent indépendamment l'un de l'autre des groupes alkyls C₈-C₂₄ ramifiés ou non ramifiés,
dans lequel la préparation d'agent oxydant contient de 5,0 à 14,0 % en poids d'hydrocarbures paraffiniques,
et
(c) de 0,01 à 2,00 % en poids d'un ou de plusieurs épaississant organiques du groupe des polysaccharides,
dans lequel la préparation d'agent oxydant contient de 0,1 à 1,0 % en poids de gomme xanthane, et
(d) 0,1 à 10,0 % d'au moins un des carbonates organiques parmi le carbonate de glycérine, le carbonate de propylène, le carbonate d'éthylène, le carbonate de diméthyle, le carbonate de diéthyle, le carbonate de di-n-hexyle, le carbonate de di-n-octyle, le carbonate de di-n-décyle et le carbonate de di-n-dodécyle sont présent dans la préparation d'agent oxydant,
avec la condition que la préparation d'agent oxydant ne contienne :
- aucun polymère qui résulterait d'une polymérisation ou d'une copolymérisation d'acide acrylique,
- aucun polymère qui résulterait d'une polymérisation ou d'une copolymérisation d'acide méthacrylique,
- aucun polymère qui résulterait d'une polymérisation ou d'une copolymérisation d'esters d'acide acrylique,
- aucun polymère qui résulterait d'une polymérisation ou d'une copolymérisation d'esters d'acide méthacrylique.

2. Préparation d'agent oxydant selon la revendication 1, **caractérisée en ce qu'**elle comprend entre 0,15 et 0,5 % en poids de gomme xanthane en tant que polysaccharide anionique (c).

3. Préparation d'agent oxydant selon l'une quelconque des revendications 1 à 2, **caractérisée en ce qu'**elle comprend également au moins entre 0,1 et 10,0 % en poids d'au moins un des solvants organiques parmi le 2-butoxyethanol, le propylène glycol, l'éther monométhylique de propylène glycol, l'éther monométhylique de diéthylène glycol, l'éther monométhylique de diéthylène glycol, l'éther monobutylique d'éthylène glycol, l'éther monoéthylique d'éthylène glycol, l'éthanol, l'isopropanol, le n-propanol, le n-butanol, le 1,2-propanediol, 1,3-propanediol, glycérine, 1-méthoxy-2-propanol, 1-éthoxy-2-propanediol, 1,4-butanediol, 1,2 hexanediol, alcool benzylique, phénoxyéthanol, alcool 2-phényléthylique, éthylène diglycol, méthyl butane et n-butylène glycol de préférence du propylène glycol et/ou de la glycérine.

4. Préparation d'agent oxydant selon l'une quelconque des revendications 1 à 3, **caractérisée en ce qu'**elle comprend entre 0,1 et 10,0 % en poids de carbonate di-n-octylique.

5. Préparation d'agent oxydant selon l'une quelconque des revendications 1 à 4, **caractérisée en ce qu'**elle comprend également au moins entre 0,1 et 10,0 % en poids d'alcool gras polyéthoxylé de formule (IV) 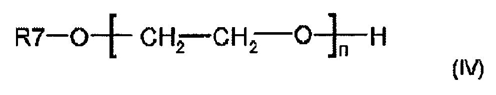
dans lequel R7 représente un groupe alkyl C₁₀-C₂₄ saturé ou non saturé et ramifié ou non ramifié ;
et
n un nombre entier compris entre 2 et 80.

6. Préparation d'agent oxydant selon l'une quelconque des revendications 1 à 5, **caractérisée en ce que** sa viscosité est comprise entre 70 et 900 mPas (22 °C sur le viscosimètre Brookfield / broche 5 / 4 Upm).

7. Préparation d'agent oxydant selon l'une quelconque des revendications 1 à 6, **caractérisée en ce qu'**elle comprend entre
(a) 0,01 et 25,0 % en poids de peroxyde d'hydrogène,
(b) 8,0 et 13,5 % en poids d'hydrocarbures paraffiniques ; et
(c) 0,1 et 1,0 % en poids de gomme xanthane.

8. Procédé pour la coloration des cheveux, **caractérisé en ce que**
(I) une préparation d'agent oxydant est parfaitement mélangée selon l'une quelconque des revendications 1 à 7 avec
(II) un agent contenant un agent d'alcalinisation et le cas échéant au moins également un précurseur de colorant par oxydation de type développeur et/ou coupleur et/ou un colorant direct,
(III) dans un godet de mélange par le fait de remuer avec un pinceau ou un applicateur,
(IV) le mélange d'application homogène fabriqué de la sorte étant appliqué sur les cheveux avec un pinceau ou un applicateur,
(V) à laisser poser entre 5 et 60 minutes sur les cheveux ; et
(VI) à rincer ensuite à l'eau ou en faisant un shampoing.
